# EUROPÄISCHE PATENTANMELDUNG

(11) **EP 4 134 973 A1**
(43) Veröffentlichungstag der Anmeldung: **15.02.2023**
(21) Anmeldenummer: 22188765.6
(22) Anmeldetag: 04.08.2022
(51) Int. Cl.: G16H 30/40

(54) **VALIDIERUNG EINES MEDIZINISCHEN SELBSTTESTS**

(30) Priorität: 12.08.2021 CH 0701562021
(71) Anmelder: Mederer GmbH, 8404 Winterthur (CH)
(72) Erfinder: Mederer, Markus, 8404 Winterthur (CH); Mederer, Deniz, 8404 Winterthur (CH)
(74) Vertreter: P&TS SA (AG, Ltd.)

(57) **Zusammenfassung**

Die Erfindung betrifft ein Verfahren und ein System zur Validierung der Durchführung eines medizinischen Testverfahrens, wobei mindestens eine Bilddatenfolge von zumindest des Probeentnahmeschrittes eines medizinischen Testverfahrens ausgeführt an einer Testperson durch ein Bildaufnahmegerät (10) an einem ersten Ort (L1) erfasst wird und die erfasste Bilddatenfolge mittels einer Sendevorrichtung an einen Verarbeitungsprozessor an einem zweiten Ort (L2) übertragen wird. Die Bilddatenfolge wird in einem der Testperson unzugänglichen Format in einer Datenbank (25) gespeichert. Mittels des Verarbeitungsprozessors wird bestimmt, ob die in der mindestens einen Bilddatenfolge erfassten vorgegebenen Schritte des Testverfahrens ordnungsgemäss durchgeführt wurden, und die Identität der Testperson auf der Grundlage von Identifikationsdaten verifiziert. Die Gültigkeitswahrscheinlichkeit eines Ergebnisses des medizinischen Testverfahrens für die Testperson wird zumindest auf Grundlage der ordnungsgemässen Durchführung der erfassten vorgegebenen Schritte des Testverfahrens und auf Grundlage der Verifizierung der Identität bestimmt.

## Beschreibung

### Technisches Gebiet

Die Erfindung betrifft eine Methode und ein System zur Überprüfung der ordnungsgemässen Durchführung eines medizinischen Testverfahrens, insbesondere eines Selbsttestverfahrens.

### Stand der Technik

Bei medizinischen Schnelltests erfolgt die Probeentnahme und Testdurchführung in der Regel ausser Haus durch medizinisch geschultes Personal. Diagnostische Schnelltests, die üblicherweise in medizinischen Einrichtungen, wie zum Beispiel Krankenhäusern, Gesundheitszentren oder Arztpraxen, durchgeführt werden sind aus der Sicht der Testperson aufwändig, da die Testperson zunächst einen Termin vereinbaren muss und sich dann in die medizinische Einrichtung begeben muss. Des Weiteren sind diese Schnelltests auch relativ teuer, da zusätzlich zu den verbrauchten Materialien auch die Leistungen der medizinischen Einrichtung und des medizinischen Personals bezahlt werden müssen. Aufgrund des notwendigen physischen Kontakts der Testperson mit anderen Personen, insbesondere während der Entnahme einer biologischen Probe für den Schnelltest an der Testperson, besteht zusätzlich auch ein Gesundheitsrisiko für alle Beteiligten.

Um den Zugang und die Durchführung von Schnelltests zu erleichtern, werden diagnostische Schnelltests zunehmend auch als Selbsttests, die unkompliziert und zu jeder Zeit zu Hause oder an einem anderen günstigen Ort ohne Assistenz von medizinisch geschulten Personen durchgeführt werden können, angeboten.

Allerdings stellt die mangelnde Nachweisbarkeit einer korrekten Ausführung eines medizinischen Selbsttests an einer Testperson ein erhebliches Problem dar. Benutzer des Selbsttests können beispielsweise Anweisungen falsch interpretieren oder auch auf Grund mangelnder Erfahrung gewisse Schritte des Tests nicht vorschriftsgemäss ausführen oder vorsätzlich versuchen zu betrügen. Eine unsachgemässe Durchführung des Testverfahrens kann zu falschen Testergebnissen, insbesondere falschen diagnostischen Aussagen, bezüglich der Testperson führen. Dies kann schwerwiegende Folgen für sowohl die Testperson, als auch für Personen, die mit der Testperson in Kontakt stehen, mit sich führen.

Im Stand der Technik sind mehrere Ansätze zur Bewertung der Durchführung von medizinischen Verfahren anhand von Bilddaten bekannt.

CN106951716A beschreibt eine telemedizinische Methode, bei der Daten der Ausführung jedes Schrittes eines Testvorgangs in Echtzeit durch ein Datenerfassungsgerät erfasst und an eine zentrale Steuereinheit übertragen werden. Die zentrale Steuereinheit verarbeitet und analysiert die Daten des Testvorgangs und überträgt das Testergebnis an ein mobiles Endgerät. Die Testperson kann daraufhin das Testresultat unter Eingabe eines korrekten Passworts abrufen. Die Vertraulichkeit der Testergebnisse ist durch den Passwort-geschützten Zugriff auf das Ergebnis gewährleistet. Allerdings ist diese Methode unzureichend, um ein erhaltenes Testergebnis mit Sicherheit einer bestimmten Person zuordnen zu können, da eine Verfälschung oder Manipulation der Methode nicht ausgeschlossen werden kann.

### Darstellung der Erfindung

Es ist ein Ziel der Erfindung, die Mängel des Stands der Technik zu beheben und eine ordnungsgemässe Durchführung eines Testverfahrens durch eine ungeschulte Person an einer Testperson zuverlässig nachzuweisen.

Es ist ein weiteres Zeil der Erfindung, dass der Nachweis einer ordnungsgemässen Durchführung eines Testverfahrens fälschungssicher erbracht wird.

Erfindungsgemäss wird dieses Ziel durch die Merkmale der unabhängigen Ansprüche erreicht.

Insbesondere wird dieses Ziel erreicht durch ein Verfahren zur Validierung der Durchführung eines medizinischen Testverfahrens, die folgenden Schritte umfassend:
- Mindestens eine Bilddatenfolge von ein oder mehreren vorgegebenen Schritten eines medizinischen Testverfahrens ausgeführt an einer Testperson wird durch ein Bildaufnahmegerät erfasst. Das Bildaufnahmegerät ist betriebsfähig mit einem Eingabegerät verbunden ist. Bildaufnahmegerät und Eingabegerät befinden sich an einem ersten Ort. Die vorgegebenen Schritte des Testverfahrens beinhalten zumindest einen Schritt einer an der Testperson ausgeführten Entnahme einer biologischen Probe.
- Die mindestens eine erfasste Bilddatenfolge wird mittels einer Sendevorrichtung an einen Verarbeitungsprozessor eines an einem zweiten Ort befindlichen Servers in Echtzeit übertragen. Der Server ist ein Computer oder ein Netzwerk von Computern.
- Die mindestens eine Bilddatenfolge wird in mindestens einer Datenbank in einem der Testperson unzugänglichen Format gespeichert. Die mindestens eine Datenbank befindet sich vorzugsweise nicht am ersten Ort. Die mindestens eine Datenbank kann sich am zweiten Ort befinden und/oder auch an einem dritten Ort befinden. Das Absichern der Bildfolge in mindestens einer Datenbank ist lediglich dadurch limitiert, dass die gespeicherte Bilddatenfolge durch die Testperson unzugänglich ist.
- Mittels des Verarbeitungsprozessors wird bestimmt, ob die in der mindestens einen Bilddatenfolge erfassten vorgegebenen Schritte des Testverfahrens ordnungsgemäss durchgeführt wurden.
- Die Identität der Testperson wird anhand von Identifikationsdaten der Testperson durch den an dem zweiten Ort befindlichen Verarbeitungsprozessors verifiziert. Vorzugsweise wird ermittelt mit welcher Wahrscheinlichkeit eine angegebene Identität der Testperson mit den erfassten Identifikationsdaten übereinstimmt.
- Die Gültigkeitswahrscheinlichkeit eines Ergebnisses des medizinischen Testverfahrens für die Testperson wird zumindest auf Grundlage der ordnungsgemässen Durchführung der erfassten vorgegebenen Schritte des Testverfahrens sowie auf Grundlage der Verifizierung der angegebenen Identität bestimmt.

Das Ziel wird des Weiteren erreicht durch ein System zur Validierung der Durchführung eines medizinischen Testverfahrens, umfassend ein an einem ersten Ort befindliches Eingabegerät, das betriebsfähig mit einem Bildaufnahmegerät und einer Sendevorrichtung verbunden ist. Des Weiteren umfasst das System einen an einem zweiten Ort befindlichen Server, der ein Computer oder ein Computer Netzwerk ist, und eine Empfangsvorrichtung, sowie mindestens einen Verarbeitungsprozessor aufweist.

Das Eingabegerät kann ein mobiles Endgerät oder ein Computer sein.

Das Bildaufnahmegerät ist beispielsweise eine Videokamera zur Aufnahme einer Bilddatenfolge in Form eines Videos.

Die Sendevorrichtung ist konfiguriert, Bilddaten in Echtzeit zu senden. Die Sendevorrichtung ist vorzugsweise eine Streaming-Vorrichtung.

Um einem Zugriff auf die gesandten Daten während deren Übertragen vorzubeugen, ist es vorteilhaft die Daten in codierter Form zu senden. Zu diesem Zweck weist die Sendevorrichtung vorzugsweise ein Mittel zur Codierung der mindestens einen Bilddatenfolge auf.

Die am zweiten Ort befindliche Empfangsvorrichtung weist ein Mittel zum Empfangen der mindestens einen Bilddatenfolge auf. Sofern die übertragenen Daten codiert wurden, ist die Empfangsvorrichtung zusätzlich mit einem Mittel zur Decodierung einer codiert übertragenen Bilddatenfolge ausgestattet.

Der mindestens eine Verarbeitungsprozessor ist dazu konfiguriert, zu bestimmen, ob die in der mindestens einen Bilddatenfolge erfassten vorgegebenen Schritte des Testverfahrens ordnungsgemäss durchgeführt wurden.

Der mindestens eine Verarbeitungsprozessor ist weiters dazu konfiguriert, die Wahrscheinlichkeit einer zutreffenden Identitätsangabe der Testperson anhand der übertragenen Bilddatenfolge oder anhand von übertragenen Identifikationsdaten, zu bestimmen.

Das System weist weiters mindestens einen Speicher zum Speichern der erfassten Bilddatenfolge in einem der Testperson unzugänglichen Format auf. Identifikationsdaten können ebenfalls in dem mindestens einen Speicher unzugänglich für die Testperson gespeichert werden.

Der mindestens eine Speicher befindet sich vorzugsweise nicht an dem ersten Ort. Der mindestens eine Speicher kann sich beispielsweise an dem zweiten Ort und/oder an einem dritten Ort befinden.

Die vorliegende Erfindung betrifft auch ein Computerprogram umfassend Befehle, die bei der Ausführung des Programms durch einen Computer diesen veranlassen, eine Gültigkeitswahrscheinlichkeit eines an einer Testperson ausgeführten medizinischen Testverfahrens auf Grund der mindestens einen erfassten Bilddatenfolge und einer bestimmten Wahrscheinlichkeit der zutreffenden Identität der Testperson zu berechnen.

Weitere vorteilhafte Ausführungsformen sind in den Unteransprüchen angegeben.

Das Validationsverfahren ist besonders für Selbsttestverfahren geeignet, die ohne Mitwirkung einer medizinisch geschulten Person ausgeführt werden. Die Testperson kann den Selbsttest an sich selbst ausführen. Es ist allerdings auch möglich das eine zweite Person das Testverfahren, insbesondere den Probeentnahmeschritt, an einer Testperson ausführt. Dies ist vor allem dann vorteilhaft, wenn die Testperson ein Kind oder eine körperlich beeinträchtigte Person ist.

In einem Aspekt der Erfindung beinhaltet das Bestimmen, ob die in der mindestens einen Bilddatenfolge erfassten vorgegebenen Schritte des Testverfahrens ordnungsgemäss durchgeführt wurden, ein Klassifizieren der erfassten Bilddatenfolge in korrekt oder in inkorrekt durchgeführte Verfahrensschritte.

Die Begriffe ordnungsgemäss und korrekt bedeuten in diesem Zusammenhang, gemäss den Anweisungen zur Ausführung des Testverfahrens der erfassten Schritte durchgeführt. Diese Anweisungen sind offizielle Anweisungen, vorzugsweise Anweisungen des Herstellers des Testkits.

In einem bevorzugten Aspekt der Erfindung erfolgt das Bestimmen, ob die in der mindestens einen Bilddatenfolge erfassten vorgegebenen Schritte des Testverfahrens ordnungsgemäss durchgeführt wurden, mittels einer künstlichen Intelligenz. Ein maschineller Lernalgorithmus kann zu diesem Zweck beispielsweise anhand bereits erfasster Datensätze der vorgegebenen Schritte des Testverfahrens trainiert werden. Der trainierte maschinelle Lernalgorithmus kann bestimmen, ob das Testverfahren korrekt durchgeführt wurde.

Das Bestimmen der ordnungsgemässen Durchführung der vorgegebenen Schritte des Testverfahrens kann synchron mit der Übertragung der mindestens einen Bilddatenfolge erfolgen. Das Bestimmen der ordnungsgemässen Durchführung der vorgegebenen Schritte des Testverfahrens kann auch asynchron erfolgen. Die asynchrone Auswertung der Bilddaten erfolgt anhand der in der Datenbank gespeicherten mindestens einen erfassten Bilddatenfolge.

Die Identifikationsdaten der Testperson werden vorzugsweise als Teil der mindestens einen Bilddatenfolge erfasst. Die Identifikationsdaten können allerdings auch separat erfasst werden.

Sofern Identifikationsdaten nicht in der mindestens einen Bilddatenfolge enthalten sind, werden diese an dem ersten Ort erfasst und in Echtzeit mittels der Sendevorrichtung an den mindestens einen an dem zweiten Ort befindlichen Verarbeitungsprozessors übertragen. Vorzugsweise werden die Identifikationsdaten mittels des Eingabegeräts erfasst.

In einem bevorzugten Aspekt dieser Erfindung wird die Identität der Testperson anhand biometrischen Identitätsdaten verifiziert. Biometrische Identitätsverifikation kann zum Beispiel auf einer automatischen Gesichtserkennung anhand des zumindest teilweise in der mindestens einen Bilddatenfolge erfassten Gesichts der Testperson beruhen. Allerdings können die Identifikationsdaten auch automatisch erfasste Fingerabdrücke oder andere physischen Eigenschaften der Testperson beinhalten.

Die Verifikation der angegebenen Identität kann punktuell oder aber auch kontinuierlich erfolgen.

Um eine Übertragung einer voraufgezeichneten Bilddatenfolge erkennen zu können, ist es von Vorteil zusätzlich die Echtzeit der Erfassung der Bilddatenfolge und/oder der Identifikationsdaten zu bestätigen. Dies kann beispielsweise anhand eines erfassten Verhaltens der Testperson in Reaktion auf unvorhersehbare auditive und/oder visuelle Signale, beispielsweise in Reaktion auf an die Testperson gerichtete Fragen und/oder Anweisungen, bestimmt werden.

In einem bevorzugten Aspekt umfasst das Validierungsverfahren des Weiteren einen Schritt des Erkennens von verfahrenstechnischen Unstimmigkeiten. Die verfahrenstechnischen Unstimmigkeiten können vorzugsweise gemäss ihrem Schweregrad gewichtet werden. Dieser Schritt kann beispielsweise das Erfassen eines zweiten Gesichts in zumindest einem Teil von Bildern der mindestens einen erfassten Bilddatenfolge beinhalten. Dieser Schritt kann das Erkennen eines vollständigen Verschwindens des Gesichts der Testperson aus zumindest einem Teil von Bildern der mindestens erfassten Bilddatenfolge beinhalten.

In einem bevorzugten Aspekt der Erfindung erfolgt die Verifizierung der angegebenen Identität nur dann kontinuierlich, sofern keine Indikation vorliegt, dass das Erfassen und die Übertragung der Identifikationsdaten nicht in Echtzeit erfolgt, und/oder sofern keine verfahrenstechnischen Unstimmigkeiten festgestellt werden.

In diesem Aspekt wird die kontinuierliche Verifikation der angegebenen Identität abgebrochen, wenn bestimmt wird, dass das Erfassen und die Übertragung der Identifikationsdaten nicht in Echtzeit erfolgt und/oder wenn eine verfahrenstechnische Unstimmigkeit erkannt wird.

Es ist weiterhin von Vorteil, der Testperson auditive und/oder visuelle Rückmeldungen, beispielsweise Warnmeldungen, Anweisungen, und/oder Fragen, geben zu können. Zu diesem Zweck kann vorzugsweise eine Benutzerschnittstelle eines mobilen Endgeräts oder eines Computers vorgesehen sein. Vorzugsweise ist die Benutzerschnittstelle Bestandteil des Eingabegeräts.

Zusätzlich zur Bilddatenfolge, die die Probeentnahme erfasst, kann auch mindestens eine Bilddatenfolge der vorgegebenen Schritte des medizinischen Testverfahren erfasst und an den Verarbeitungsprozessor übertragen werden. Es kann auch mindestens eine Bilddatenfolge der Aufbereitung und/oder der Applikation der entnommenen biologischen Probe erfasst und übertragen werden. Diese weiteren Bilddatenfolgen können mittels des Verarbeitungsprozessors ausgewertet und in einer Datenbank in einem der Testperson unzugänglichen Format gespeichert werden.

Das medizinische Testverfahren kann beispielsweise ein diagnostischer in vitro Test sein. Das Testverfahren kann beispielsweise ein Antigentest, ein Antigenschnelltest, ein Antikörpertest, ein Antikörperschnelltest, ein Pulse Controlled Amplification (PCA)-Schnelltest, oder ein Polymerase Chain Reaction (PCR)-Schnelltest sein.

Das biologische Probematerial kann beispielsweise Schleimhautzellen, Saliva, Sputum, oder Kapillarblut beinhalten.

Die biologische Probe kann der Testperson beispielsweise nasal, nasopharyngeal, oropharyngeal oder durch Punktion der Fingerbeere entnommen werden. Die Probeentnahme kann beispielsweise mittels eines Abstrichbestecks, einer Lanzette oder durch Spucken der Testperson, erfolgen. Ein Abstrichbesteck ist beispielsweise ein Abstrichtupfer, eine Abstrichbürste oder ein Abstrichspatel.

Beispielsweise kann die Probeentnahme folgende Schritte umfassen:
- Einführen eines Abstrichbestecks in die nasale, nasopharyngeale, oder oropharyngeale Öffnung einer Testperson, sodass der zur Probegewinnung bestimmte Abschnitt des Abstrichbestecks eine vorgegebene Stelle, vorzugsweise die Rachenwand der Testperson, kontaktiert;
- Aufrechterhalten des Kontaktes des zur Probegewinnung bestimmten Abschnitts des Abstrichbestecks mit der vorgegebenen Stelle für eine vorbestimmte Zeitspanne;
- Optional, Ausführen eines vorgegebenen Bewegungsablaufs, beispielsweise einer Drehbewegung, an der vorgegebenen Stelle;
- Nach Ablauf der vorbestimmten Zeitspanne, Entfernen des Abstrichbestecks aus der nasalen oder nasopharyngealen Öffnung einer Testperson.

Die mindestens eine erfasste Bilddatenfolge wird für die Testperson unzugänglich in einer Datenbank gespeichert. Der Begriff unzugänglich bedeutet in diesem Zusammenhang, dass die Testperson die unzugänglichen Daten nicht abrufen kann. Eine Manipulation der Daten oder eine mögliche Fälschung der Datenfolge durch die Testperson wird somit vermieden.

Um einer Manipulation der erfassten Daten vorzubeugen, ist es zusätzlich von Vorteil, wenn ein Zugriff auf die in einer Datenbank gespeicherten Bilddatenfolgen und/oder Identifikationsdaten, ausschliesslich für autorisierte Personen möglich ist. Eine Zugriffsautorisation sollte vorzugsweise beantragt und überprüft werden müssen, bevor die Autorisation erteilt wird. Vorzugsweise erfolgt die Identifikation der autorisierten Person für den Zugriff auf die Datenbank anhand biometrischer Erkennung. Die Identifikation der autorisierten Person kann auf einer zwei-Schritt-Identifikation basieren.

Vorzugsweise ist es für autorisierte Personen möglich, die gespeicherten Daten zu kommentieren oder auszuwerten. In dieser Ausführung ist es weiters von Vorteil, wenn Kommentare und/oder Auswertungsergebnisse der autorisierten Personen in der Datenbank gespeichert werden können. Optional können autorisierte Personen Testergebnisse und Kommentare an die Testperson übermitteln. Es kann auch wünschenswert sein, dass autorisierte Personen eine Kopie der erfassten Bilddatenfolgen oder Identifikationsdaten an die Testperson übertragen können.

Diese Kopie der Bilddatenfolge kann zum Beispiel zusammen mit Kommentaren oder Verbesserungsvorschlägen zur korrekten Durchführung des bewerteten Verfahrens an die Testperson übermittelt werden. Allerdings muss in jedem Fall gewährleistet sein, dass ein Zugriff der Testperson auf die in der Datenbank gespeicherten Originaldaten nicht möglich ist.

Vorzugsweise beruht das Bestimmen der Gültigkeitswahrscheinlichkeit eines Ergebnisses des medizinischen Testverfahrens für die Testperson neben der ordnungsgemässen Durchführung der erfassten vorgegebenen Schritte des Testverfahrens und der Verifizierung der angegebenen Identität zusätzlich auch auf der Abwesenheit oder Anwesenheit von vorzugsweise gewichteten verfahrenstechnischen Unstimmigkeiten und/oder dem Verifizieren der Echtzeit der Erfassung der Bilddatenfolge.

### Kurze Beschreibung der Figuren

Die Erfindung wird anhand der in den Figuren dargestellten möglichen Ausführungsbeispielen näher erläutert, wobei
**Figur 1** schematisch Komponenten eines möglichen Ausführungsbeispiels der Erfindung, illustriert;
**Figur 2** ein Ablaufdiagramm eines exemplarischen Prozesses an einem ersten Ort L1 ist;
**Figur 3** ein Ablaufdiagramm eines exemplarischen Prozesses des Verfahrens beim Start des Datenstroms an einem zweiten Ort L2 mit optionaler Validierung in Echtzeit ist;
**Figur 4** ein Ablaufdiagramm eines exemplarischen parallelen Prozesses der Validierung innerhalb des Verfahrens am zweiten Ort L2 ist;
**Figur 5** ein Ablaufdiagramm eines exemplarischen parallelen Prozesses der Validierung der Identifikationsdaten innerhalb des Verfahrens am zweiten Ort L2 ist;
**Figur 6** ein Ablaufdiagramm eines exemplarischen parallelen Prozesses der Validierung der Identifikationsdaten zur biometrischen Betrugserkennung, vorzugsweise mittels maschinellen Lernens, innerhalb des Verfahrens am zweiten Ort L2 ist;
**Figur 7** ein Ablaufdiagramm eines exemplarischen Prozesses der Validierung der Identifikationsdaten zur biometrischen Verifikation der Testperson, vorzugsweise mittels maschinellen Lernens, innerhalb des Verfahrens am zweiten Ort L2 ist;
**Figur 8** ein Ablaufdiagramm eines exemplarischen parallelen Prozesses der Durchführung innerhalb der Validierung ist.

### Wege zur Ausführung der Erfindung

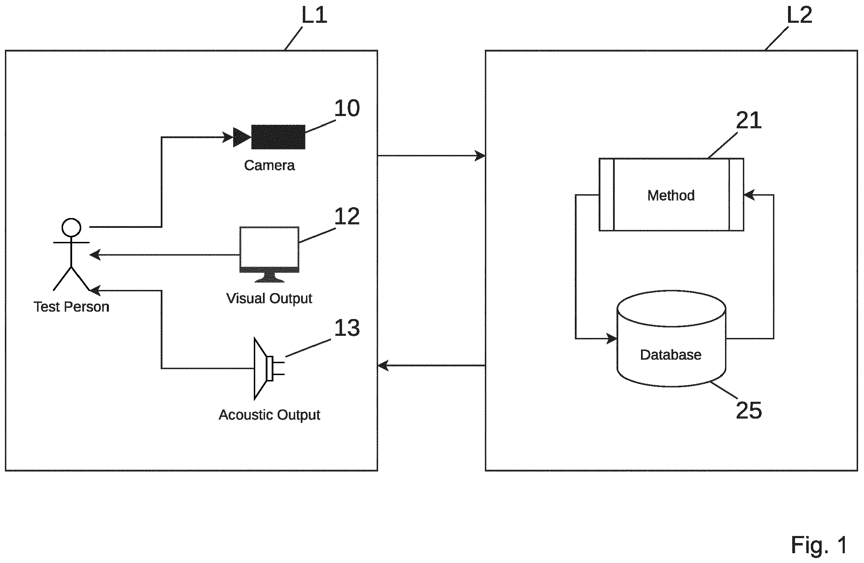

Erfindungsgemässe Ausführungsbeispiele sind in den Figuren 1 bis 8 dargestellt und nachstehend beschrieben.

Die räumliche Verteilung der verschiedenen Komponenten des Systems zur Validierung der ordnungsgemässen Durchführung des Testverfahrens, wird in Figur 1 schematisch anhand eines Ausführungsbeispiels der Erfindung veranschaulicht.

Die Testperson, an der die Probeentnahme des medizinischen Verfahrens ausgeführt wird, sowie das Bildaufnahmegerät 10, das beispielsweise eine Videokamera ist, befinden sich an einem ersten Ort L1.

Das Bildaufnahmegerät ist an ein Eingabegerät (nicht abgebildet) betriebsfähig angeschlossen. Das Bildaufnahmegerät kann in dem Eingabegerät integriert sein. Das Eingabegerät ist beispielsweise ein mobiles Endgerät oder ein Computer. Das Bildaufnahmegerät kann allerdings auch als separate Einheit betriebsfähig mit dem Eingabegerät verbunden sein. Das Bildaufnahmegerät ist dazu ausgestattet, die Bilddatenfolge von vorgegebenen Schritten eines medizinischen Testverfahren ausgeführt an einer Testperson an dem ersten Ort zu erfassen. Die erfasste Bilddatenfolge beinhaltet zumindest den Verfahrensschritt der Probeentnahme.

Die erfasste Bilddatenfolge wird mittels einer Sendevorrichtung (nicht abgebildet) an eine an einem zweiten Ort L2 befindliche Empfangsvorrichtung (nicht abgebildet) übertragen und dort an einen Verarbeitungsprozessor gegeben. Empfangsvorrichtung und Verarbeitungsprozessor sind Bestandeilte eines an dem zweiten Ort befindlichen Servers. Der Verarbeitungsprozessor führt ein Computer-implementiertes Verfahren 21 aus, das den Prozessor veranlasst, anhand der mindestens einen Bilddatenfolge die ordnungsgemässe Durchführung der erfassten Verfahrensschritte des Testverfahrens zu bestimmen.

Die Verifizierung von erfassten Identitätsdaten, sowie die Gültigkeitswahrscheinlichkeit der Ergebnisse des Testverfahrens werden ebenfalls mittels eines Computer-implementierten Verfahrens, das durch den Verarbeitungsprozessor ausgeführt wird, ermittelt.

Bilddatenfolgen sowie Identitätsdaten werden in diesem Ausführungsbeispiel in einer an dem zweiten Ort L2 befindlichen Datenbank 25, die hier ebenfalls Bestandteil des Servers ist, gespeichert.

Die Methode 21 beruht vorzugsweise auf Maschinellem Lernen. Um den maschinellen Lernalgorithmus zu trainieren, können voraufgezeichnete Datensätze beispielsweise von korrekten Bewegungsabläufen verwendet werden. Anhand der in der Datenbank 25 gespeicherten und durch den Verarbeitungsprozessor klassifizierten Bilddatenfolgen kann der maschinelle Lernalgorithmus weiters kontinuierlich trainiert werden.

Die übertragenen Daten können entweder synchron, das heisst zum Zeitpunkt der Datenübertragung, oder asynchron ausgewertet werden. Zur asynchronen Auswertung werden die übertragenen Daten in der Datenbank 25 gespeichert. Die gespeicherten Daten können dann zu einem späteren Zeitpunkt ausgewertet werden.

Rückmeldungen bezüglich der Auswertung des Testverfahrens, Anweisungen und/oder Informationen können mittels einer in dem Server enthaltenen Sendevorrichtung an eine Empfangsvorrichtung an dem ersten Ort übertragen werden. Mittels einer visuellen Ausgabe 12 und/oder einer akustischen Ausgabe 13, die an ein Ausgabegerät (nicht abgebildet) betriebsfähig angeschlossen, oder Bestandteil des Ausgabegeräts sind, werden diese daraufhin der Testperson übermittelt.

Die nachfolgenden Figuren zeigen Ablaufdiagramme einzelner Verfahrensschritte möglicher Ausführungsbeispiele.

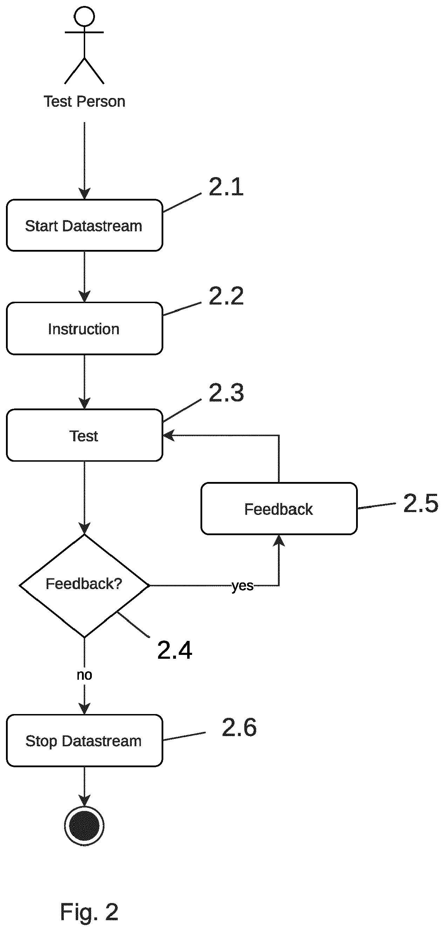

Figur 2 ist ein Ablaufdiagramm eines exemplarischen Prozesses am ersten Ort L1. Die Testperson ist anwesend und löst den Datenstrom 2.1 aus. Die Testperson erhält daraufhin Anweisungen 2.2. Die Anweisungen dienen beispielsweise dazu, die Testperson in der ordnungsgemässen Ausführung des Tests zu instruieren.

Die Anweisungen können allerdings auch dazu dienen, die Echtzeit einer Bilddatenfolge zu bestätigen. So kann eine unvorhersehbare Anweisung die Testperson dazu veranlassen, etwas zu tun, das bildlich oder akustisch erfasst und maschinell ausgewertet wird. Dadurch wird ermittelt, ob der Test zum Zeitpunkt der Bilderfassung durchgeführt wird, oder ob die übertragene Bilddatenfolge voraufgezeichnet ist. Im letzteren Fall wird die Durchführung des Tests abgebrochen werden, da höchstwahrscheinlich eine betrügerische Tätigkeit vorliegt.

Zwecks der Bestätigung der Echtzeit der Bilddatenfolge sollten derartige unvorhersehbare Anweisung sowohl bei einer synchronen, als auch bei einer asynchronen Auswertung der Bilddatenfolge an die Testperson ausgegeben werden.

Es ist beispielsweise möglich, eine Vielzahl möglicher Anweisungen vorzudefinieren und zum Zeitpunkt der Testdurchführung eine spezifische Anweisung zufällig aus dieser Vielzahl auszuwählen, sodass diese spezifische Anweisung für die Testperson nicht vorhersehbar ist. Die Anweisung sollten nicht situationsabhängig sein.

Anweisungen und Erfassen des Verhaltens der Testperson können beispielsweise auf dem Prinzip eines biometrischen Captcha («*Completely Automated Public Turing test* to *teil Computers and Humans Apart»*) Modells beruhen.

Die Anweisungen können von einem Prozessor, oder aber auch von einer Person ausgegeben werden. Der anweisende Prozessor oder die anweisende Person befindet sich nicht am ersten Ort L1. Beispielsweise können die Anweisungen von dem am zweiten Ort L2 befindlichen Verarbeitungsprozessor ausgegeben werden.

In einem weiteren Schritt wird der medizinische Test 2.3 durchgeführt, wobei zumindest die Entnahme der biologischen Probe an der Testperson mittels eines Bildaufnahmegeräts und der Sendevorrichtung in Echtzeit übertragen wird.

Optional erstellt ein Verarbeitungsprozessor auch eine Rückmeldung bezüglich der Durchführung des Tests 2.4. Diese Rückmeldung wird an die Testperson übermittelt 2.5. Allerdings ist dieser Rückmeldungsschritt nur in synchron ausgewerteten Verfahren möglich.

Im Falle einer negativen Rückmeldung ist es möglich, dass der Test oder einzelne Testschritte nochmals durchgeführt werden müssen.

Wenn die Durchführung der vorbestimmten Schritte des Testverfahrens beendet ist, wird die Echtzeit-Übertragung der Bilddatenfolge angehalten 2.6.

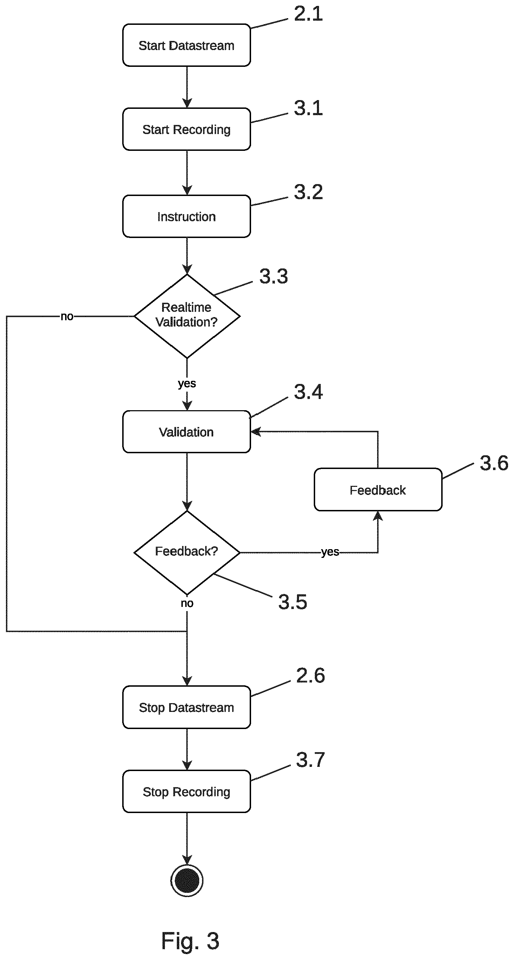

Figur 3 zeigt ein Ablaufdiagram eines exemplarischen Prozesses des Verfahrens beim Start des Datenstroms 2.1 am zweiten Ort L2 mit optionaler Validierung der ordnungsgemässen Durchführung der vorgegebenen Schritte des Testverfahrens in Echtzeit. Der übertragene Datenstrom löst eine elektronische Aufzeichnung der übertragenen Bildfolgedaten aus 3.1. In dem angeführten Beispiel gibt der am zweiten Ort L2 befindlichen Verarbeitungsprozessor eine Anweisung an die am ersten Ort L1 befindlichen Testperson aus 3.2. Die Anweisung wird mittels einer geeigneten Sender- und Empfängervorrichtung übertragen. Anhand dieser Anweisungen wird, wie oben erwähnt, vorzugsweise die Echtzeit der übertragenen Bildfolge bestätigt.

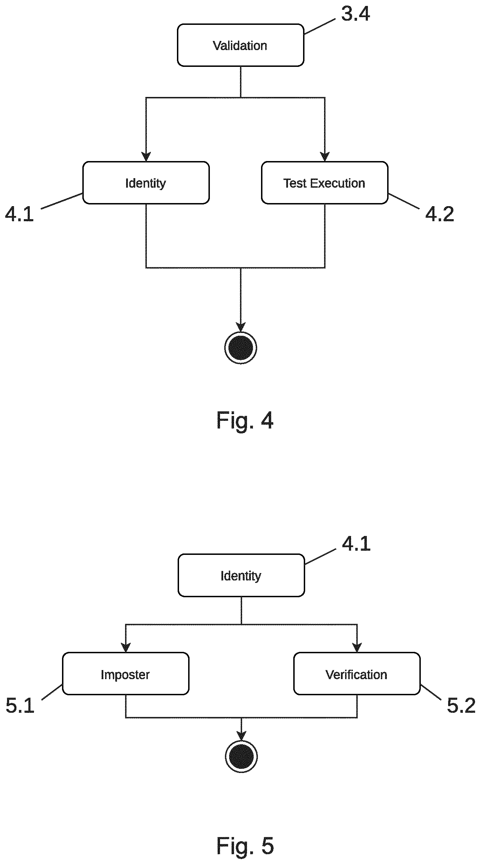

In einem nächsten Schritt wird entschieden, ob eine Validierung in Echtzeit durchgeführt werden soll 3.3. Die Validierung der erhaltenen Daten betrifft sowohl die Durchführung des Testverfahrens als auch die Bestätigung der Identität der Testperson, wie in der nachfolgenden Figur 4 beschrieben wird. Falls die Echtzeit der Übertragung nicht bestätigt werden kann, wird der Datenstrom sofort abgebrochen 2.6. Mit dem Anhalten der Echtzeit-Übertragung der Bilddatenfolge wird die Datenaufnahme beendet 3.7.

Folgend eines positiven Entscheids bezüglich der Echtzeit, wird ein Computer-implementiertes Validationsverfahren 3.4 durch den Verarbeitungsprozessor ausgeführt.

Optional kann der Verarbeitungsprozessor eine Rückmeldung bezüglich der Durchführung des Tests erstellen 3.5, welche an die Testperson übermittelt wird 3.6.

Gemäss Figur 4 beruht das Validationsverfahren 3.4 dieses Ausführungsbeispiels auf der Bestätigung der Identität 4.1 der Testperson, sowie der korrekten Durchführung des Testverfahrens 4.2. Die Bestätigung der Identität erfolgt vorzugsweise kontinuierlich. Diese Bestätigung kann aber auch punktuell oder einmalig erfolgen.

Eine Verifikation einer für die Testperson angegebene Identität erfolgt auf Grund von Identifikationsdaten, die bereits in der übertragenen Bilddatenfolge enthalten sein können. In einem bevorzugten Ausführungsbeispiel berechnet der Verarbeitungsprozessor einen Wahrscheinlichkeitswert für die Übereinstimmung der angegebenen Identität mit den erfassten Identifikationsdaten.

Figur 5 veranschaulicht, dass die Berechnung der Wahrscheinlichkeit der zutreffenden Identität der Testperson, sowohl auf einer Verifikation der angegebenen Identität 5.2, als auch auf der Abwesenheit von verfahrenstechnischen Unstimmigkeiten, die darauf hinweisen, dass es sich bei der in zumindest einem Teil der übertragenen Bildfolge erfassten Person um eine andere Person als die Testperson 5.1 handelt, beruht.

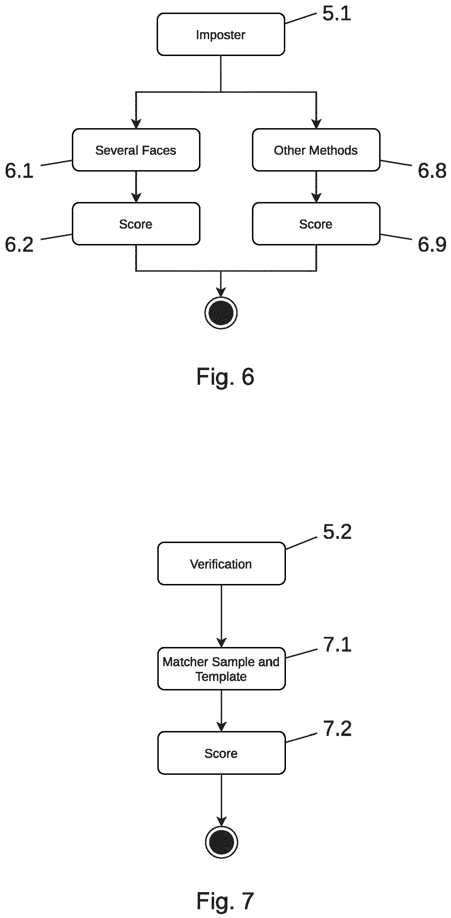

In Figur 6 wird verdeutlicht, dass diese verfahrenstechnischen Unstimmigkeiten beispielsweise durch das Erscheinen eines unvorhergesehenem zweiten Gesichts 6.1 in zumindest einem Teil der übertragenen Bilddatenfolge erkannt werden. Allerdings können auch andere oder zusätzliche Methoden 6.8 basierend auf anderen Hinweisen auf Unstimmigkeiten zu diesem Zweck ausgeführt werden. Vorzugsweise wird auf Grund der erfassten Unstimmigkeiten ein gewichteter Wert 6.2, 6.9 bestimmt, der in die Bestimmung der Wahrscheinlichkeit der korrekt angegebenen Identität der Testperson miteinbezogen werden kann.

Figur 7 veranschaulicht einen exemplarischen Schritt zur Validation der übertragenen Identifikationsdaten zur biometrischen Verifikation der Testperson. Die Identität der Testperson wird mittels Vorlagedaten, zum Beispiel Identifikationsdaten die als biometrisches Merkmal einer Identität zugeordnet werden, angegeben. Die Vorlagedaten werden an den Verarbeitungsprozessor übermittelt. Der Verarbeitungsprozessor gleicht die Vorlagedaten mit den übertragenen Identifikationsdaten, die in den Bildfolgedaten enthalten sein können, ab 7.1. Die Übereinstimmung der Vorlagedaten mit den Identifikationsdaten wird ermittelt und ein Überstimmungswert 7.2 berechnet.

Dieser Übereinstimmungswert kann zur Bestimmung der Wahrscheinlichkeit der zutreffenden Identität der Testperson miteinbezogen werden.

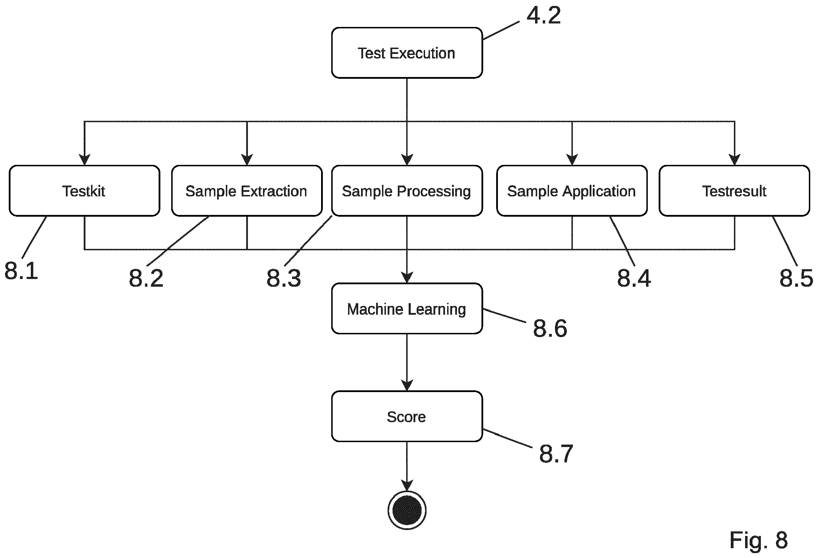

Figur 8 ist ein Ablaufdiagram, das exemplarisch parallel-verlaufende Prozesse der Durchführung des beanspruchten Validierungsverfahrens veranschaulicht. Eine parallele Auswertung der erfassten Datensätze erfolgt vorzugsweise durch maschinelles Lernen, zum Beispiel mittels eines trainierten neuronalen Netzwerks.

Die übertragenen Daten bezüglich des Testverfahrens werden mittels des Verarbeitungsprozessors an dem zweiten Ort L2 validiert 4.2.

Zur Beurteilung der Validität eines Testverfahrens und/oder zur Bestimmung der Validität eines Resultats desselben Testverfahrens, kann die Validität folgender Datensätze miteinbezogen werden:
- die Authentizität eines Testkits 8.1, beispielsweise mittels einer bildlichen Erfassung der Testkit Nummer;
- die ordnungsgemässe Durchführung der Probeentnahme 8.2, beispielsweise mittels einer bildlichen Erfassung einer farblichen Veränderung des Abstrichbestecks;
- die ordnungsgemässe Durchführung der Probeaufbereitung 8.3, beispielsweise mittels einer bildlichen Erfassung einer farblichen Veränderung des Aufbereitungsmaterials;
- die ordnungsgemässe Durchführung der Applikation der Probe in dem Test 8.4;
- die Eindeutigkeit des beispielsweise bildlich erfassten Ergebnisses des Tests 8.5.

Die diesbezüglichen Datensätze können parallel, das heisst gleichzeitig und gemeinsam, von einem maschinellen Lernalgorithmus ausgewertet werden. Es ist allerdings auch möglich, dass die einzelnen Datensätze auch separat ausgewertet werden und erst nachträglich zur Bestimmung eines Gültigkeitswertes oder einer Gültigkeitswahrscheinlichkeit 8.7 kombiniert werden.

Es versteht sich, dass verschiedene Ausführungen und Modifikationen des hierin beschriebenen Verfahrens möglich und für die Fachperson offensichtlich sind. Soweit diese Ausführungsformen nicht vom Umfang der Ansprüche abweichen, ist es vorgesehen, dass diese ebenfalls in der hier offengelegten Erfindung enthalten sind.

## Patentansprüche

1. Verfahren zur Validierung der Durchführung eines medizinischen Testverfahrens, umfassend
- Erfassen mindestens einer Bilddatenfolge von ein oder mehreren vorgegebenen Schritten eines medizinischen Testverfahrens ausgeführt an einer Testperson durch ein Bildaufnahmegerät (10), das betriebsfähig mit einem Eingabegerät verbunden ist, an einem ersten Ort (L1), wobei die vorgegebenen Schritte des Testverfahrens zumindest einen Schritt einer an der Testperson ausgeführten Entnahme einer biologischen Probe umfasst;
- Übertragen in Echtzeit der mindestens einen erfassten Bilddatenfolge mittels einer Sendevorrichtung an einen Verarbeitungsprozessor eines an einem zweiten Ort (L2) befindlichen Servers, der ein Computer oder ein Computer Netzwerk ist;
- Speichern der mindestens einen Bilddatenfolge in mindestens einer Datenbank (25) in einem der Testperson unzugänglichen Format;
- Bestimmen mittels des Verarbeitungsprozessors, ob die in der mindestens einen Bilddatenfolge erfassten vorgegebenen Schritte des Testverfahrens ordnungsgemäss durchgeführt wurden;
- Verifizieren der Identität der Testperson auf der Grundlage von Identifikationsdaten mittels des an dem zweiten Ort (L2) befindlichen Verarbeitungsprozessors;
- Bestimmen der Gültigkeitswahrscheinlichkeit eines Ergebnisses des medizinischen Testverfahrens für die Testperson zumindest auf Grundlage der ordnungsgemässen Durchführung der erfassten vorgegebenen Schritte des Testverfahrens und auf Grundlage der Verifizierung der Identität.

2. Das Validierungsverfahren nach Anspruch 1, wobei die Identität der Testperson auf Grundlage biometrischer Identifikationsdaten, die in der mindestens einen Bilddatenfolge enthalten sind, verifiziert wird.

3. Das Validierungsverfahren nach einem der Ansprüche 1 oder 2, wobei das Bestimmen der ordnungsgemässen Durchführung der in der mindestens einen Bilddatenfolge erfassten vorgegebenen Schritte mittels einer künstlichen Intelligenz, die anhand bereits erfasster Datensätze der vorgegebenen Schritte des Testverfahrens trainiert wurde, erfolgt.

4. Das Validierungsverfahren nach einem der Ansprüche 1 bis 3, wobei die Identität der Testperson kontinuierlich verifiziert wird.

5. Das Validierungsverfahren nach einem der Ansprüche 1 bis 4, wobei verfahrenstechnische Unstimmigkeiten in der mindestens einen erfassten Bilddatenfolge bestimmt werden.

6. Das Validierungsverfahren nach einem der Ansprüche 4 und 5, wobei die kontinuierliche biometrische Verifizierung der Identität nur durchgeführt wird, solange keine verfahrenstechnischen Unstimmigkeiten festgestellt werden.

7. Das Validierungsverfahren nach einem der Ansprüche 1 bis 6, wobei eine oder mehrere auditive und/oder visuelle Rückmeldungen mittels einer Benutzerschnittstelle des Eingabegeräts an die Testperson ausgegeben werden.

8. Das Validierungsverfahren nach einem der Ansprüche 1 bis 7, wobei die biologische Probe der Testperson nasal, oropharyngeal, nasopharyngeal, oder durch Punktion, beispielsweise der Fingerbeere, entnommen wird.

9. Das Validierungsverfahren nach einem der Ansprüche 1 bis 8, wobei ein Zugriff auf die mindestens eine in einer Datenbank (25) des Servers gespeicherten Bilddatenfolgen, ausschliesslich für autorisierte Personen möglich ist.

10. Das Validierungsverfahren nach einem der Ansprüche 1 bis 9, wobei das medizinische Testverfahren ein Selbsttestverfahren ist, das die Testperson an sich selbst ausführt.

11. Ein System zur Validierung der Durchführung eines medizinischen Testverfahrens, umfassend
- ein an einem ersten Ort (L1) befindliches Eingabegerät, das betriebsfähig verbunden ist mit:
∘ einem Bildaufnahmegerät (10) zum Erfassen mindestens einer Bilddatenfolge von vorgegebenen Schritten eines medizinischen Testverfahren ausgeführt an einer Testperson an dem ersten Ort;
∘ einer Sendevorrichtung zum Übertragen der mindestens eine erfassten Bilddatenfolge in Echtzeit; und
- mindestens einen Speicher zum Speichern der erfassten Bilddatenfolge in einem der Testperson unzugänglichen Format;
- einen am zweiten Ort (L2) befindlichen Server, der ein Computer oder ein Computer Netzwerk ist, umfassend:
∘ eine Empfangsvorrichtung mit einem Mittel zum Empfangen der mindestens einen Bilddatenfolge;
∘ mindestens einen Verarbeitungsprozessor, der dazu konfiguriert ist, zu bestimmen, ob die in der mindestens einen Bilddatenfolge erfassten vorgegebenen Schritte des Testverfahrens ordnungsgemäss durchgeführt wurden, und der weiters dazu konfiguriert ist, die Wahrscheinlichkeit einer zutreffenden Identitätsangabe der Testperson anhand der übertragenen Bilddatenfolge oder anhand von übertragenen Identifikationsdaten, zu bestimmen.

12. Computerprogramm umfassend Befehle, die bei der Ausführung des Programms durch einen Computer diesen veranlassen zu bestimmen, ob die in der nach dem ersten Anspruch übertragenen mindestens einen Bilddatenfolge die erfassten vorgegebenen Schritte des Testverfahrens ordnungsgemäss durchgeführt wurden.
